# EUROPEAN PATENT SPECIFICATION

(11) **EP 4 000 590 B1**
(45) Date of publication and mention of the grant of the patent: **22.05.2024**
(21) Application number: 21203919.2
(22) Date of filing: 21.10.2021
(51) Int. Cl.: A61J 15/00

(54) **FEEDING TUBE**
ERNÄHRUNGSSONDE
TUBE D'ALIMENTATION

(30) Priority: 19.11.2020 GB 202018212
(43) Date of publication of application: 25.05.2022
(73) Proprietor: GBUK Group Limited, Selby, North Yorkshire YO8 5DD (GB)
(72) Inventor: COWAN, Joseph, Selby, YO8 5DD (GB); ALLSOPP, Ross, Selby, YO8 5DD (GB); HAINDL, Cornelia, Selby, YO8 5DD (GB)
(74) Representative: Murgitroyd & Company

(56) References cited:
- US-A1- 2014 114 259
- US-A1- 2020 086 107

## Description

The present invention relates to a feeding tube, and in particular to a gastric feeding (gastrostomy) tube (*eg* a G-tube).

Adequate nutrition and medication are essential for patients recovering from surgery, illness or suffering from chronic conditions or congenital problems. In many cases, patients may be unable to suck or swallow and are therefore unable to take adequate nutrients or medicines orally. In these situations, patients may be fitted with a gastrostomy tube (also known as a feeding tube, gastric feeding tube or G-Tube). The gastrostomy tube provides a means to introduce a nutrient and/or medication directly into the stomach.

Gastrostomy tubes are usually fitted to a patient through percutaneous endoscopic gastrostomy (PEG). A gastroscopy is performed to determine the location and anatomy of the stomach. This is typically performed by transillumination using a light source on an endoscope within the stomach. An incision is then made in the abdominal wall and a guidewire is inserted and then pulled out of the mouth. The gastrostomy tube is then attached to the guide wire and pulled in through the mouth, oesophagus and stomach and then out through the incision. In certain cases the gastrostomy tube may be positioned surgically. Once positioned, the gastrostomy tube remains in position and a stoma tract is established through the abdominal wall and into the stomach.

Once fitted, gastrostomy tubes may remain in position for a variable amount of time depending on the needs of the patient. For short-term recovery or respite the tube may only remain in place for a few days or weeks. For patients experiencing long-term chronic or congenital conditions such as problems of the mouth, oesophagus, stomach or intestines the tube may remain in place for several months or longer and may be replaced multiple times.

A conventional gastrostomy tube is typically a flexible tube composed of silicone or polyurethane. A bolster is positioned at the internal end of the tube *(i.e.* the end intended to be positioned within the stomach). A bolster helps to retain the tube in position whilst sealing the stomach wall. In recent times balloons tend to be used on replacement gastrostomy tubes in preference to bolsters due to ease of removal. Balloon gastrostomy tubes have a bulb which can be inflated once inside the stomach (using sterile water or another fluid) to prevent the tube being pulled out through the stoma tract. The balloon can be deflated again when the tube needs to be removed.

US2014/114259A1 discloses a conventional balloon gastrostomy tube with a detachable feeding tube extension. US2020/086107A1 discloses a balloon gastrostomy tube with separable internal and external portions.

To enable *in situ* inflation and deflation of the balloon, the tube requires two channels or lumens: a main channel to carry a nutrient and/or medicine into the stomach (or to drain the contents of the stomach) and a secondary channel to carry fluid to inflate the balloon. It is important to ensure that these channels remain separate to prevent stomach fluids escaping, or premature deflation (or over-inflation) of the balloon. The two channels (or lumens) are usually positioned within the same tube and are separated by an internal wall.

In addition to the balloon (or bolster), an external retention device is positioned on an external portion of the tube (*i.e.* a portion positioned or intended to be positioned outside the body of the patient). In use, the external retention device is positioned against the patient's abdomen. It supports the tube and prevents the tube from being drawn further into the stomach. Together with the balloon (or bolster) within the stomach, the external retention device limits longitudinal movement of the tube once in place.

Balloon gastrostomy tubes can be replaced by simply deflating the balloon and then pulling the tube out through the stoma tract. A new tube with a deflated balloon can then be inserted through the stoma tract and the balloon inflated once inside the stomach. This simple procedure can often be carried out at home without assistance from a clinician. In comparison, gastrostomy tubes with rigid bolsters or bumpers must be removed by a clinician.

The external end of the tube (*i.e.* the end intended to be positioned outside the body) typically has a Y-port. The Y-port has two connection ports: a feed connection port to allow food or medicine supplies such as a feeding bag to be connected to the main channel; and a balloon inflation port to allow a pressurised fluid supply to be connected to the secondary channel to inflate or deflate the balloon.

The Y-port is typically secured to the external end of the tube via an adhesive and/or by mechanical attachment. Bonding a silicone tube to a hard plastic Y-port is not straightforward: the low relative surface energy of silicone rubber means that it does not readily interact with adhesives or other surfaces. Chemical alteration to improve adhesion of the silicone is not necessarily an option for medical grade silicone tubing as it must remain bioinert and not cause irritation to the patient. Typically the tubing at the connection to the Y-port has to be textured (*e.g.* with a *"Christmas tree "* type connection) used in combination with a specific adhesive. This attachment is a common cause of tube failure.

The connection between the tubing and the Y-port may become loose over time, leading to leaks or a loss of balloon pressure. The Y-port itself may also become damaged or require changing to enable attachment of different or updated devices. Regular manipulation of the Y-port is necessary to attach, change and remove medicine or nutrient supplies. The Y-port may fail as a result of repeated pulling, tugging or snagging on clothes. The seals or threads may become damaged through wear after repeated use. This wear may be exacerbated through contact with corrosive stomach acids, particularly if the gastrostomy tube is used to drain stomach contents.

Given the complexity of the connection between the tube and the Y-port, it is not possible to fix the connection or replace the Y-port without removing and replacing the entire gastrostomy tube. This increases costs, results in more wastage and is inconvenient for the patient.

Often a clamp is placed on the external portion of the tube between the external retention device and the Y-port. The clamp can be used to close the main channel of the tube to prevent gastric fluids escaping during (for example) changing of the feeding bag. However the clamp also closes the secondary channel. This can cause the pressure of fluid within the balloon to increase, potentially leading to failure of the balloon. The clamp can also damage the internal wall separating the two channels of the tube over prolonged and repeated use. Failures of the internal wall and balloon are not possible to rectify without removing and replacing the entire gastrostomy tube, again resulting in increased costs and wastage as well as inconvenience and discomfort to the patient.

The present invention seeks to improve the performance of gastrostomy tubes, in particular by reducing the risk of component failure.

Viewed from a first aspect the present invention provides a gastrostomy tube for enteral nutrition or medication of a subject, comprising:
a first flexible tube having an internal end positionable *in vivo* within the stomach of the subject and an external end positionable *ex vivo,* wherein the first flexible tube has a main longitudinal channel and a secondary longitudinal channel which are separated and are not in fluid communication with each other;
an internal retention device attached at or towards the internal end of the first flexible tube and in fluid communication with the secondary longitudinal channel;
a substantially Y-shaped connector attached to the external end of the first flexible tube, wherein the substantially Y-shaped connector has a first port, a second port and a third port, wherein the external end of the first flexible tube is connected to the first port, the second port is in fluid communication with the main longitudinal channel and the third port is in fluid communication with the secondary longitudinal channel; and
a second flexible tube having a first end and a second end and a conduit extending between the first end and the second end, wherein the first end is bonded with a silicone adhesive to the second port of the substantially Y-shaped connector to define a delivery channel extending between the internal end of the first flexible tube and the second end of the second flexible tube for delivering a nutrient or medicine to the subject, wherein the second end of the second flexible tube is attachable to a nutrient or medicine source.

The substantially Y-shaped connector may be a connector having three arms which extend radially from a juncture. The three arms may be substantially coplanar. The three arms may have the same length or different lengths. The arms may extend substantially equiangularly from the juncture. For example, each of the angles between adjacent arms may be about 120 °. Alternatively two of the three angles between adjacent arms may be the same or the three angles may be different.

Preferably an angle between a first arm (having the first port) and a second arm (having the second port) is about 180 ° (*ie* the first and second ports are substantially coaxial).

Preferably an angle between the first arm and a third arm (having the third port) is larger than an angle between the second arm and the third arm.

The gastrostomy tube of the present invention has the substantially Y-shaped connector at an intermediate position along the delivery channel remote from an outer terminus of the delivery channel at the second end of the second flexible tube. This configuration offers several advantages. The Y-shaped connector is positioned closer to the body of the patient and is less likely to be damaged through snagging or pulling. By positioning the Y-shaped connector at an intermediate position along the delivery channel, the length of the first flexible tube can be reduced. This decreases manufacturing costs because a shorter length of the more complex, expensive dual lumen tube is required. As only the first flexible tube (and the internal retention device) is intended to be positioned within the stoma tract and stomach, this means that a smaller proportion of the gastrostomy tube must be made of high-grade material suitable for use *in vivo.*

By providing a second flexible tube, a feed connection port (which may be attached to the second end of the second flexible tube) can be longitudinally distanced from the Y-shaped connector. In use, this reduces the need to manipulate the Y-shaped connector as it is only actively used when inflating or deflating the balloon. This reduces the risk of failure of the Y-shaped connector and in particular the connection between the first flexible tube and the first port of the Y-shaped connector. If a feed connection port fails (for example as a result of repeated use), it can be removed from the second flexible tube and replaced without having to replace the entire gastrostomy tube.

Typically the conduit is a single conduit.

The second flexible tube provides a convenient site for clamping or otherwise shutting off flow along the delivery channel without the need to clamp the dual channel (lumen) first flexible tube.

The optimum length of the first flexible tube will vary depending on the patient. Larger patients will typically require a longer first flexible tube. As the second flexible tube is positioned entirely *ex vivo* its length is not dependent on the size of the patient. The first flexible tube may therefore be longer than, the same length as or shorter than the second flexible tube. Preferably the first flexible tube is between 5 cm and 15 cm in length. Preferably the length of the first flexible tube is minimised, *i.e.* the length is equal to or up to 2 cm longer than the length of the patient's stoma tract. This reduces the required amount of high grade material suitable for *in vivo* use.

Preferably a longitudinal distance between the substantially Y-shaped connector and the second end of the second flexible tube is equal to or greater than a longitudinal distance between the substantially Y-shaped connector and the internal retention device. In the context of this invention, "longitudinal distance" should be interpreted as a distance measured along the delivery channel *(i.e.* the main channel of the first flexible tube, an intermediate channel extending between the first and second ports of the Y-shaped connector and the conduit of the second flexible tube).

Preferably the second flexible tube is at least 5 cm in length. Even more preferably the second flexible tube is between 8 cm and 12 cm in length This provides spare material to allow the second flexible tube to be cut to a shorter length as required, and/or a new or replacement feed connection port to be attached.

The size (external diameter) of the first flexible tube will vary depending on the specific needs of the patient. Preferably the first flexible tube has a French size of between 10 and 22 inclusive.

Preferably a cross-sectional area of the main longitudinal channel is larger than a cross-sectional area of the secondary longitudinal channel. Preferably the cross-sectional area of the main longitudinal channel is between 2 and 20 times larger than the cross-sectional area of the secondary longitudinal channel. More preferably the cross-sectional area of the main longitudinal channel is between 7 and 12 times larger than the cross-sectional area of the secondary longitudinal channel.

Preferably the cross-sectional area of each of the main and secondary longitudinal channels remains substantially constant along the length of the first flexible tube.

The main longitudinal channel may have any suitably shaped cross-section to enable the transfer of the nutrient or medicine in liquid, suspension, paste or pulp form. Preferably the main longitudinal channel has a substantially circular, oval or substantially D-shaped cross-section. A substantially circular cross-section reduces the risk of blockages through the build-up of material in corners. A substantially D-shaped cross-section balances the advantages of the circular cross-section whilst ensuring an adequate minimum thickness of an internal separating wall between the main and secondary longitudinal channels.

Preferably the secondary longitudinal channel has a substantially circular cross-section. A substantially circular cross-section ensures that pressure forces from the balloon inflation fluid are distributed more evenly. This minimises forces exerted on the internal separating wall.

Preferably a cross-sectional area of the conduit is the same size or larger than the cross-sectional area of the main longitudinal channel. Where the cross-sectional area of the conduit is larger than the cross-sectional area of the main longitudinal channel, the intermediate channel of the Y-shaped connector extending between the second and first ports preferably tapers to gradually adjust the diameter of the delivery channel between the second and first flexible tubes. This helps to limit blockages.

The gastrostomy tube may further comprise a feed connection port attached to the second end of the second flexible tube. The feed connection port may be attached to the second flexible tube permanently. For example the feed connection port may be attached to the second flexible tube using an adhesive or a physical connector such as a *"Christmas-tree* " type connector, a friction fit or a clamp or grip-type connector. Where a permanent connection is used, the feed connection port can be replaced by cutting the second flexible tube to remove the feed connection port, and then fitting a new feed connection port using adhesive or a physical connection to the cut end of the second flexible tube. The feed connection port is preferably compatible with ENFit type connectors.

The first flexible tube and the substantially Y-shaped connector are preferably composed of a first material and the second flexible tube is preferably composed of a second material which is different from the first material. The first material is preferably silicone of a grade suitable for use *in vivo.* By forming the substantially Y-shaped connector and the first flexible tube from the same material, an appropriate adhesive can be selected to improve the strength of the bond between the first port and the first flexible tube. The first flexible tube may be connected to the Y-shaped connector by an adhesive and/or a physical connector such as a *"Christmas tree* " type connector or friction fit. Alternatively the first flexible tube and the substantially Y-shaped connector may be formed as a single continuous part. The second material is preferably polyurethane.

The gastrostomy tube may further comprise an external retention device adjustably mounted on the first flexible tube. The external retention device may be mounted to be adjustable longitudinally along the length of the first flexible tube. The external retention device may be mounted permanently (*i.e*. so it cannot be removed from the first flexible tube without breaking or cutting) or it may be mounted removably. The external retention device may be composed of a rigid plastic (such as polyvinyl chloride) or may be composed of a flexible material such as polyurethane or silicone. Preferably the external retention device is composed of a thermoplastic polyurethane.

The external retention device may retain its longitudinal position on the first flexible tube frictionally (for example through a tight friction fit around the circumference of the first flexible tube). Alternatively the external retention device may have a locking mechanism which is operable to grip the first flexible tube and prevent movement of the external retention device along the first flexible tube.

Preferably the external retention device has a main body and a locking arm hingedly connected at or near to the perimeter of the main body. The main body may have an aperture at or near to an end through which the first flexible tube may be snugly fitted. The first flexible tube may pass through a duct extending substantially perpendicularly away from the aperture along the main body. The duct may at least partially enclose the first flexible tube. By virtue of the aperture and duct, the external retention device may be slidable longitudinally along the first flexible tube to allow selective positioning.

Preferably the duct has a substantially circular cross-section of a size suitable to receive snugly the first flexible tube. Preferably the duct has an elongate slot in an outer wall. The first flexible tube may be able to pass through the aperture and the duct.

Preferably the locking arm is pivotal in an arc between an open position and a closed position. The closed position may be locked.

Preferably in the closed position, a locking rod on the free end of the locking arm is captured within a correspondingly-shaped groove on the main body.

The inner wall of the locking arm preferably has one or more teeth configured to pass through the elongate slot to contact and grip the first flexible tube positioned within the duct. This serves to prevent longitudinal movement of the first flexible tube in the locked position.

The gastrostomy tube may further comprise a clamp mounted to the second flexible tube. The clamp may be operable to shut off a fluid pathway between the first and second ends of the second flexible tube. By providing the clamp on the second flexible tube, an internal end of the delivery channel is selectively isolable from the feed connection port (for example when no nutrient or medicine source is connected). This prevents leakage of stomach acids through the feed connection port which could damage the seals and threads on the feed connection port as well as being unpleasant for the subject.

The clamp is advantageously mounted exclusively on the second flexible tube. By avoiding clamping the first flexible tube, the likelihood of the internal separating wall of the first flexible tube failing is reduced.

Preferably the internal retention device comprises a balloon having an elastic body with an interior void in fluid communication with the secondary longitudinal channel. The balloon may be inflated by passing fluid through the third port and secondary channel into the interior void. The balloon may be composed of a suitable material such as silicone. The fluid is preferably a liquid such as sterile water or a saline solution. The fluid may be applied from a syringe attached to the third port. The third port may be configured to attach directly a syringe. For example the third port may be provided with a thread which is engageable with a corresponding thread on the nozzle of a syringe. Alternatively a separate adapter may be attached to the third port to enable a syringe to be attached.

Viewed from a second aspect the present invention provides a kit of parts comprising: a gastrostomy tube as hereinbefore defined; and a nutrient/medicine container attachable or attached to the gastrostomy tube via the second flexible tube or the feed connection port.

The kit of parts may further comprise one or more adapters attachable to the second end of the second flexible port or the feed connection port. The adapter may permit various nutrient/medicine containers, syringes or other items to be connected.

The kit of parts may further comprise a syringe adapter attachable to the third port. The syringe adapter may permit a syringe to be attached to the third port.

The kit of parts may further comprise a first syringe attachable to the third port to supply fluid to and/or to remove fluid from the internal retention device. The first syringe may be preloaded with a predetermined quantity of sterile fluid. This prevents errors by ensuring that the internal retention device is charged with a sufficient quantity of fluid to prevent it being pulled out through the stoma tract of the subject prematurely, whilst avoiding over inflation of the balloon.

The kit of parts may further comprise a second syringe attachable to the delivery channel via the second flexible tube or the feed connection port. This second syringe advantageously enables the stomach contents of the patient to be periodically tested (for example pH measurement) or a test to be performed prior to removing and replacing a gastrostomy tube.

A specific implementation of the present invention will now be described, by way of example only, and with reference to the accompanying drawings in which:
**Figure 1** is a perspective view of a gastrostomy tube according to a first embodiment of the present invention.
**Figure 2** is a longitudinal cross-sectional view of the gastrostomy tube of Figure 1.
**Figure 3** is an expanded view of the Y-shaped connector of Figure 2.
**Figure 4** is an expanded view of the Y-shaped connector, first flexible tube and internal retention device of Figure 2.
**Figure 5** is an expanded view of the internal retention device of Figure 4.
**Figure 6** is an expanded view of the Y-shaped connector, second flexible tube and feed connection port of Figure 2.
**Figure 7** is a transverse cross-sectional view of the first flexible tube and internal retention device of Figure 1, viewed looking towards the internal end.
**Figure 8** is a perspective view of the first flexible tube and internal retention device of Figure 1.
**Figure 9** is a simplified cross-sectional view of the gastrostomy tube of Figure 1 in position in the abdomen of a patient, with the external retention device in an open state.
**Figure 10** is a perspective view of the gastrostomy tube of Figure 1 including an external retention device and a clamp.
**Figure 11** is a longitudinal side view of the gastrostomy tube of Figure 10.
**Figure 12** is a longitudinal side view of the gastrostomy tube of Figure 11 showing the balloon inflated.
**Figure 13** is a perspective view of the external retention device of Figure 10 in an open position.
**Figure 14** is a different perspective view of the external retention device of Figure 13 in an open position.

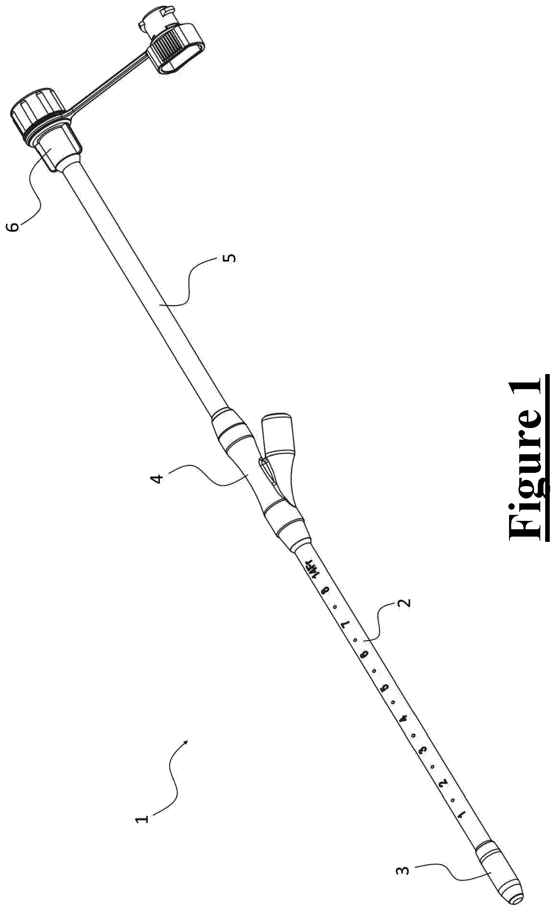

Referring initially to Figure 1, a gastrostomy tube 1 of a first embodiment of the invention is shown. The gastrostomy tube 1 has a first flexible tube 2 connected at an internal end to an internal retention device 3 and at an external end to a Y-shaped connector 4. A second flexible tube 5 is also connected at a first end to the Y-shaped connector 4 and has a feed connection port 6 attached at a second end.

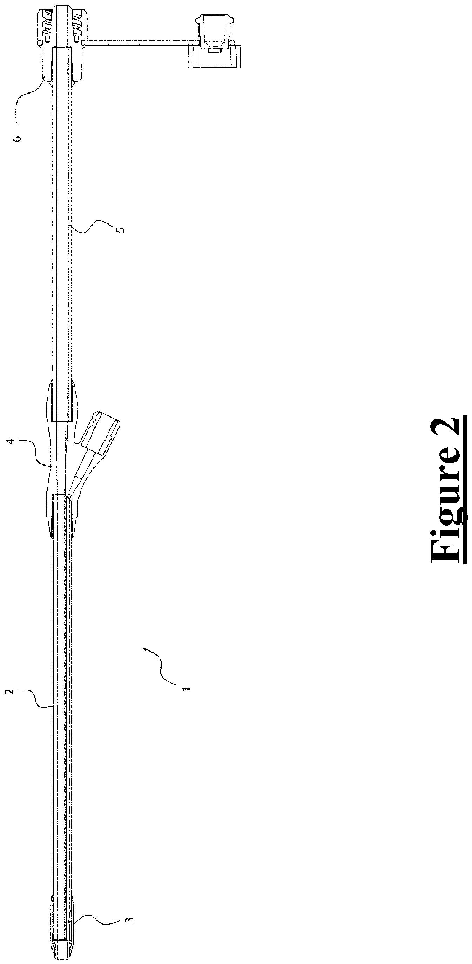

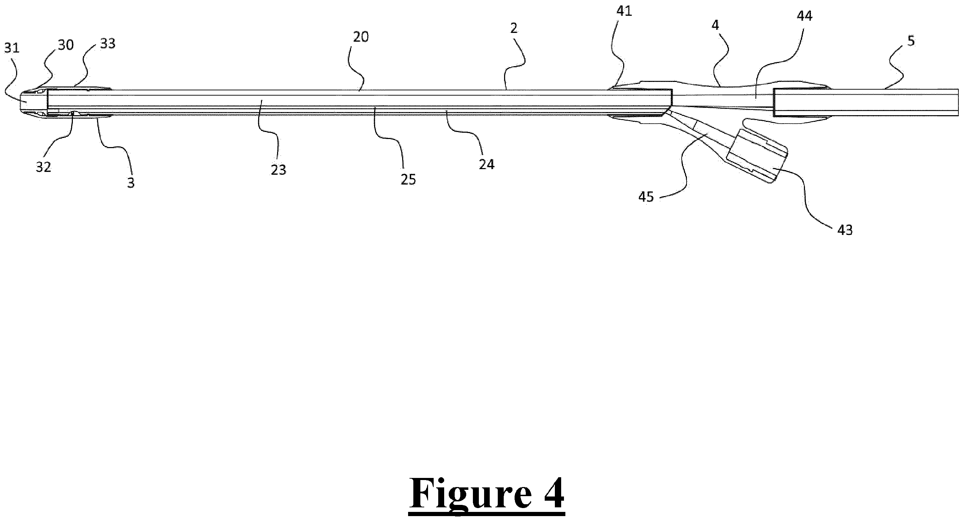

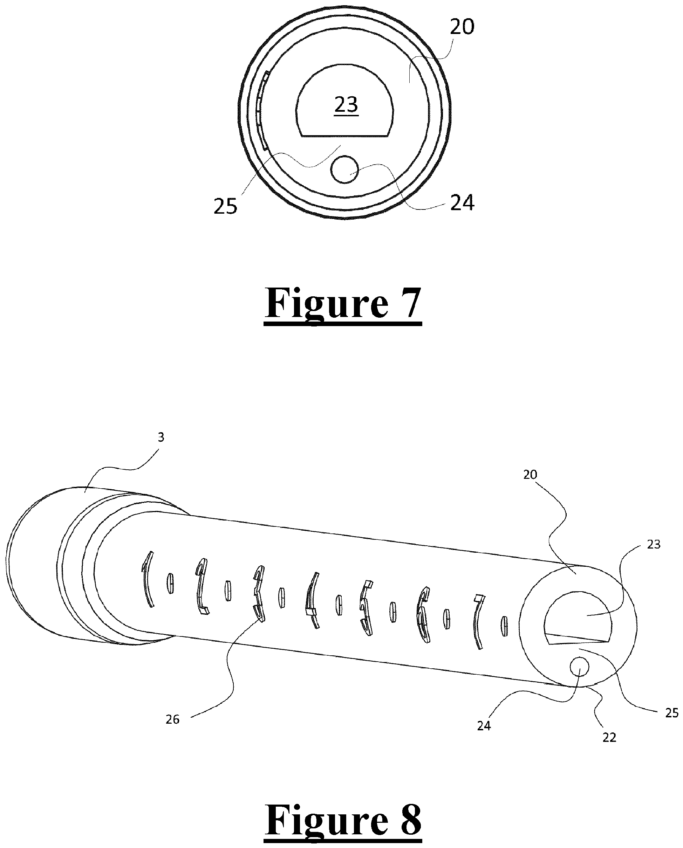

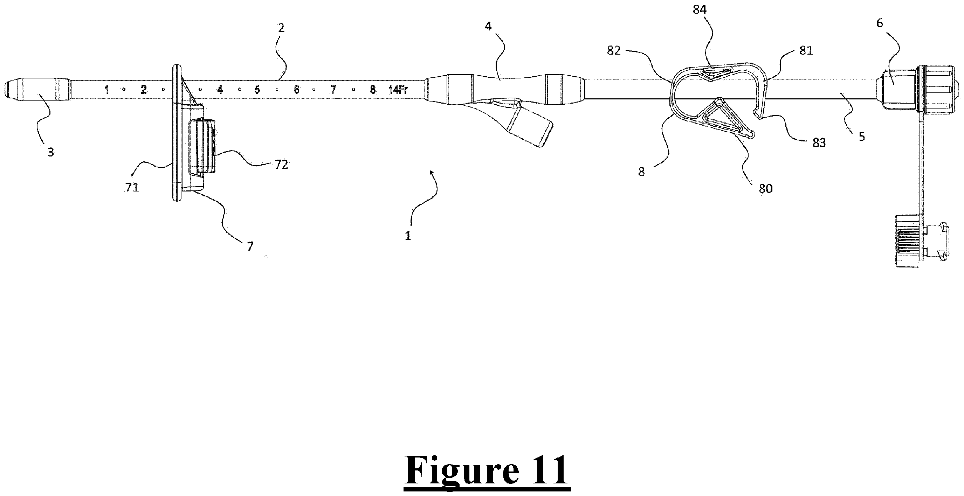

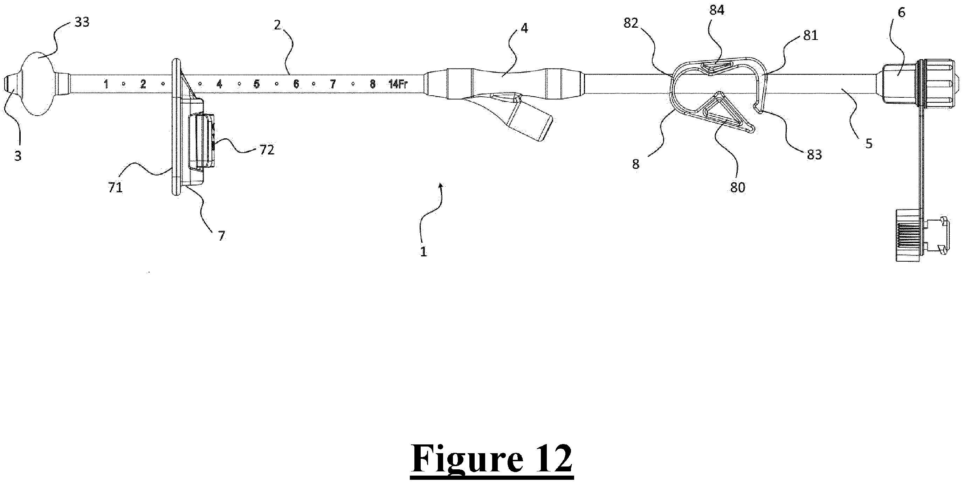

As best illustrated by Figures 2, 4, 7 and 8, the first flexible tube 2 is a dual lumen or dual channel tube having a main channel 23 and a secondary channel 24 running substantially parallel to the main channel 23. Both channels are surrounded by an external tubing wall 20. The two channels are separated by a separating wall 25. In this embodiment, the main channel 23 has a substantially D-shaped cross-section and the secondary channel 24 has a circular cross-section. The main channel 23 has a larger cross-sectional area than the secondary channel 24. The cross-sectional areas of the main and secondary channels 23, 24 remains essentially the same along the entire length of the first flexible tubing 2. An external surface of the first flexible tube 2 is marked with a gradated scale 26 to measure tract length to ensure correct placement of the gastrostomy tube 1 within the abdomen of a patient. The gradated scale 26 shown in Figures 1, 8 and 10-12 measures the tract length in centimetres. The external diameter of the first flexible tube 2 is also marked on the external surface to ensure the correct diameter tubing is used. In this embodiment the first flexible tube 2 has a French size of 14. The first flexible tube 2 is composed of medical grade silicone.

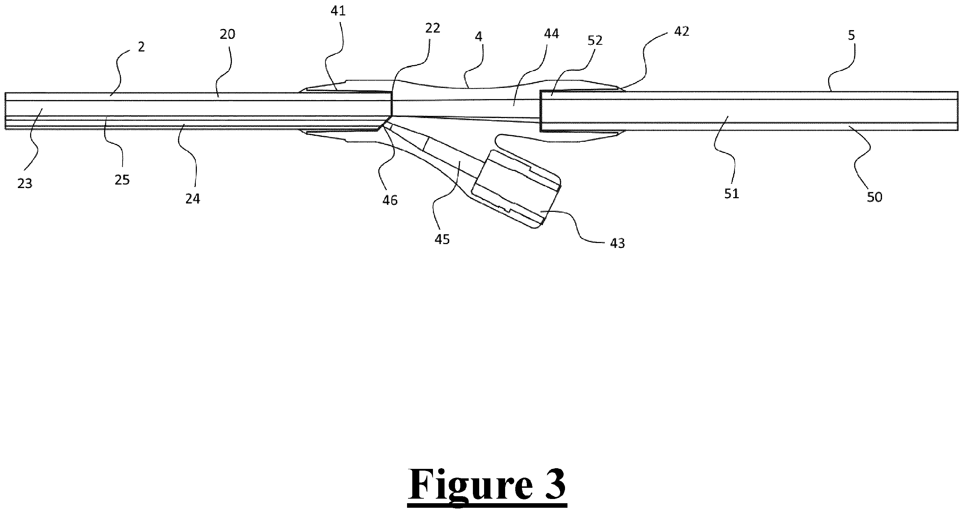

Referring now to Figure 3, a longitudinal cross-section of the Y-shaped connector 4 is shown. The Y-shaped connector 4 has a first port 41, a second port 42 and a third port 43. In this embodiment, the first and second ports 41, 42 are disposed along a common longitudinal axis, whilst the third port 43 is offset with respect to the common longitudinal axis. The first and second ports 41, 42 are connected by an intermediate channel 44 which runs along the longitudinal axis between the second port 42 and the first port 41. The first and third ports 41, 43 are connected by an inflation channel 45.

Each of the first, second and third ports 41, 42, 43 are shaped and sized differently to ensure correct assembly. The third flexible port 43 may be fitted with a suitable adapter (not shown) to enable attachment of a syringe. The first port 41 has a protruding wedge 46 which corresponds to a cutting in the external end 22 of the first flexible tube 2. The inflation channel 45 opens into the first port 41 at the protruding wedge 46. This ensures correct alignment of the first flexible tube 2 in the first port 41 so that the secondary channel 24 is aligned and in fluid communication with the inflation channel 45 and the main channel 23 is aligned and in fluid communication with the intermediate channel 44. The separating wall 25 and body of the Y-shaped connector 4 ensure that the intermediate channel 44 and main channel 23 are completely separated from and are not in fluid communication with the secondary channel 24 and the inflation channel 45. The Y-shaped connector 4 is composed of medical grade silicone. The external end 22 of the first flexible tube 2 is bonded to the first port 41 with a silicone adhesive.

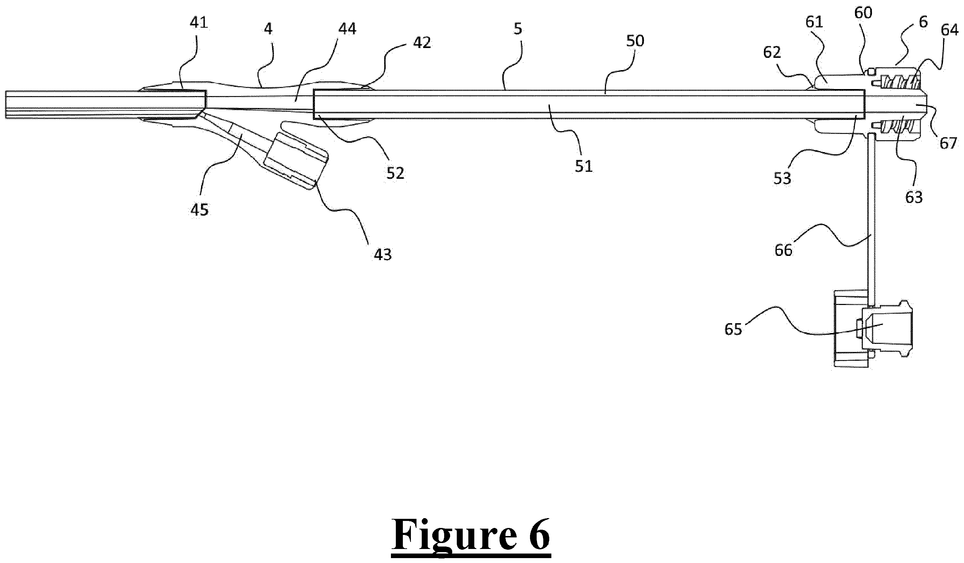

Referring again to Figure 3 and to Figure 6, a second flexible tube 5 is connected at a first end 52 to the second port 42. The second flexible tube 5 is composed of polyurethane. As the second flexible tube 5 is positioned entirely *ex vivo* in normal use there is no need for it to be composed of expensive medical grade silicone. The first end 52 is bonded to the second port 42 with a silicone adhesive. The second flexible tube 5 is a single lumen tube and consists of a single conduit 51 extending between the first end 52 and the second end 53 and surrounded by an outer wall 50. The conduit 51 has a circular cross-section. The conduit 51 is in fluid communication with the intermediate channel 44, the main channel 23 and an opening 31 of the internal retention device 3 to define a delivery channel extending along the full length of the gastrostomy tube.

The second flexible tube 5 has the same outer diameter as the first flexible tube 2. However, as the first flexible tube 2 has two internal channels and the second flexible tube 5 only has one internal channel, the cross-sectional area of the main channel 23 is smaller (and a different shape) to the cross-sectional area of the conduit 51. The intermediate channel 44 of the Y-shaped connector 4 is therefore tapered to gradually adjust the diameter and shape of the delivery channel. This gradual transition between the larger circular cross-section of the conduit 51 and the smaller substantially D-shaped cross-section of the main channel 23 aids the smooth movement of fluids along the delivery channel and prevents pressure build-ups or blockages.

As best illustrated by Figure 6, a feed connection port 6 is connected to the second end 53 of the second flexible tube 5. The feed connection port 6 has a body 60 with a female port 61 and a male port 63. The female port 61 receives the second end 53 of the second flexible tube 5. The second flexible tube 5 is bonded to the female port 61 using an adhesive 62. The male port 63 has a protruding central tube 67 which is in fluid communication with the conduit 51. Arranged around the central tube 67 is a thread 64. The thread 64 allows a nutrient or medicine source to be connected via a threaded connector to the delivery channel via the central tube 67.

The feed connection port 6 also has a cap 65 which can be screwed into the male port 63 to seal the open end of the central tube 67 and delivery channel. This may be advantageous to prevent backflow and leakage of stomach acids through the delivery channel when, for example, no nutrient or medicine source is connected to the gastrostomy tube 1. The cap 65 is attached to the main body 60 of the feed connection port 6 via a flexible arm 66.

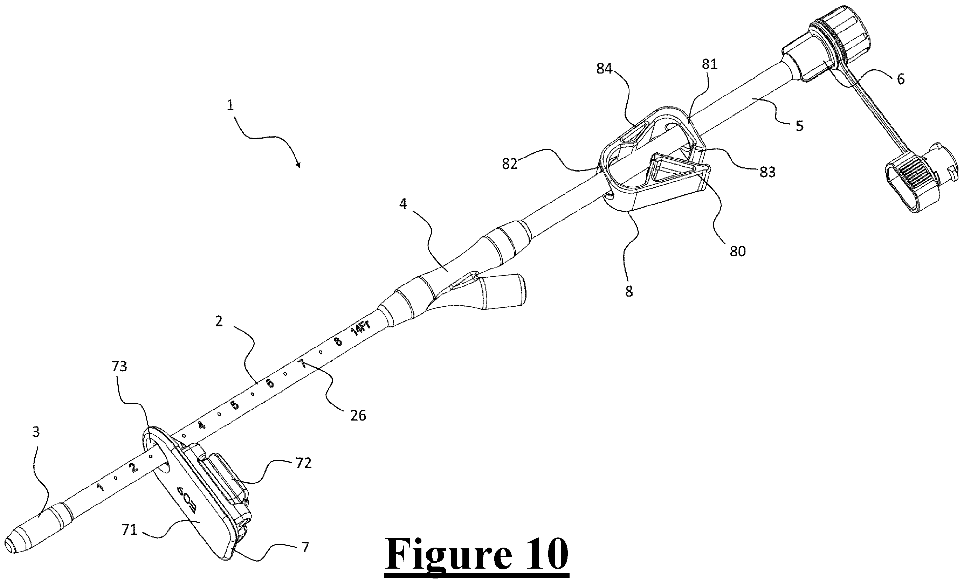

Referring now to Figures 10-14, the gastrostomy tube 1 is also equipped with an external retention device 7. The external retention device 7 has a main body 70 and an aperture 73 in the main body 70 through which the first flexible tube 2 is snugly fitted. The first flexible tube 2 passes through a duct 74 in the central part of the main body 70. The duct 74 extends substantially perpendicularly to the aperture 73. The duct 74 has an elongate slot in an outer wall and therefore only partially encloses the first flexible tube 2. The external retention device 7 is slidable longitudinally along the first flexible tube 2 to allow selective positioning.

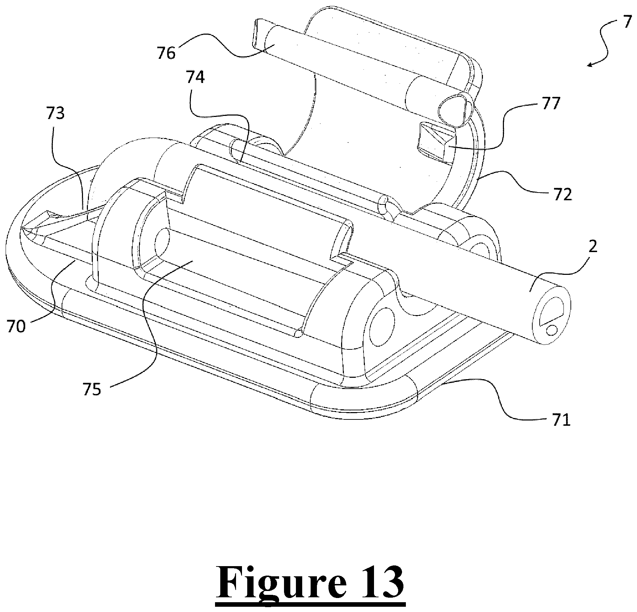

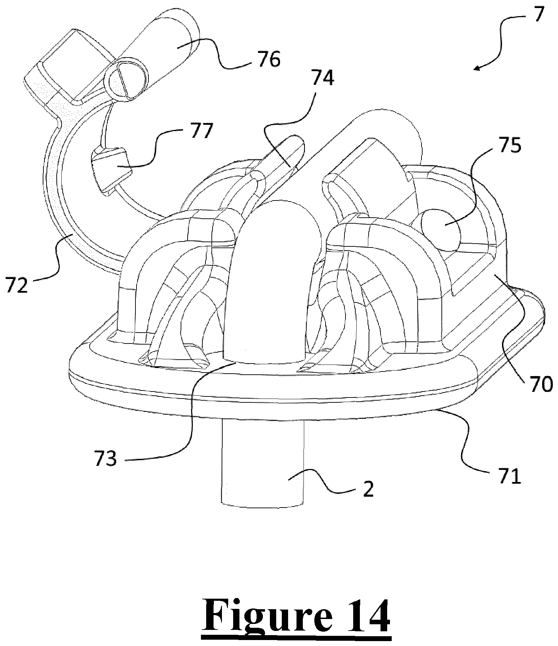

A locking arm 72 is hingedly connected to the perimeter of the main body 70. The locking arm is pivotal in an arc between an open position (shown in Figures 13 and 14) and a locked position (not shown). In the open position, the first flexible tube 2 is removable from the duct 74 through the elongate slot to leave the external retention device 7 retained on the first flexible tube 2 only via the aperture 73. In the locked position, a locking rod 76 on the free end of the locking arm 72 is captured within a correspondingly-shaped groove 75 on the main body 70. A tooth 77 on an inner wall of the locking arm 72 passes through the slot of the duct 74 and grips the first flexible tube 2 to prevent longitudinal movement of the first flexible tube 2. The grip provided by the tooth 77 in the locked position is tight enough to hold the first flexible tube 2 but not so tight that the first flexible tube 2 is compressed as this could block the main and/or secondary channels 23, 24. An underside 71 of the main body 70 is configured to be substantially planar to sit against the skin of the patient in use. The external retention device 7 is composed of a thermoplastic polyurethane.

The gastrostomy tube 1 also has a clamp 8 positioned around the second flexible tube 5. The clamp 8 provides a convenient means to isolate the feed connection port 6 from the rest of the gastrostomy tube 1, for example when a nutrient or medicine source is attached or removed from the feed connection port 6. This prevents stomach acid backflowing up the main channel 23 and conduit 51 and leaking.

The clamp 8 has a first shoulder 81 and a second shoulder 82. The two shoulders are displaced longitudinally relative to each other at either end of a main body 84 and each have an aperture through which the second flexible tube 5 is passed. The second shoulder 82 has an arm 80 which is resiliently biased in an open position (shown) and can be moved into a locked position (not shown) to press the second flexible tube 5 against the main body 84 to close the conduit 51 and prevent fluid flow. The first shoulder 81 is also resiliently deformable and has a protrusion 83 which acts to retain the arm 80 in the locked position as required. The arm 80 can be released from the locked position by pulling the first shoulder 81 outwards (*i*.*e*. away from the second shoulder 82).

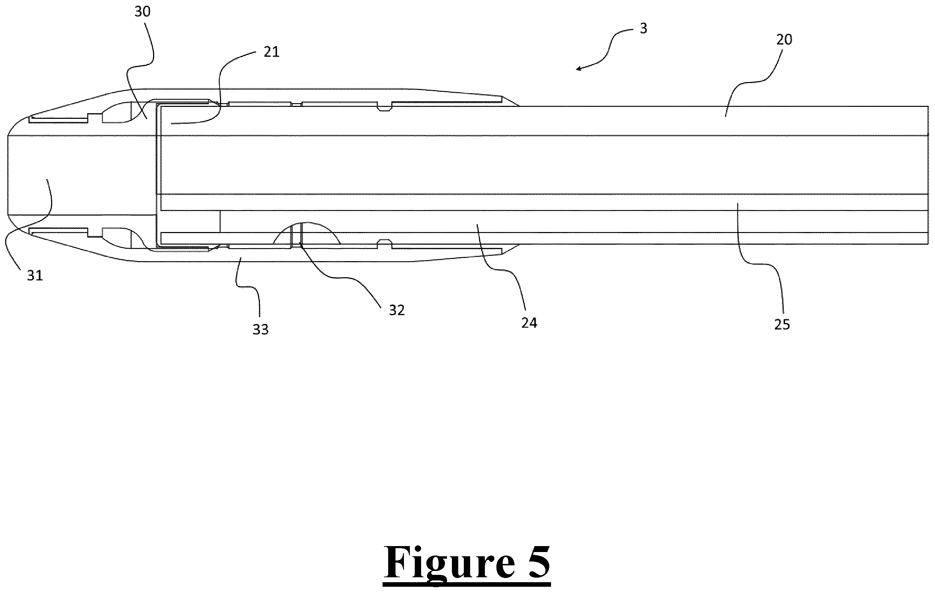

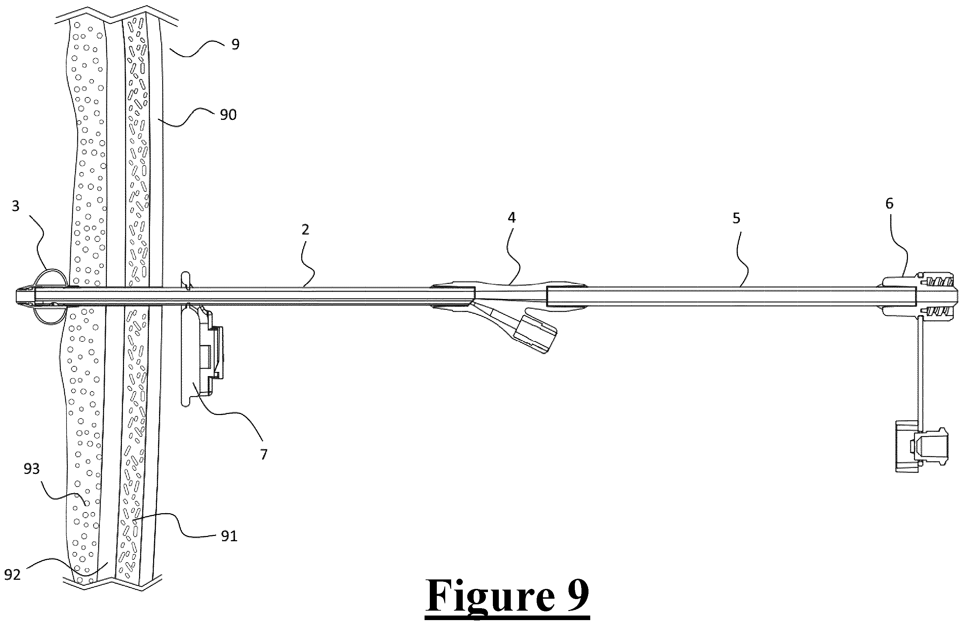

A patient being fitted with gastrostomy tube 1 will typically already have an established stoma tract owing to an earlier fitted gastrostomy tube or PEG procedure. As shown in Figure 9, the stoma tract is a channel established through the layers of skin 90, fatty tissue 91, muscle tissue 92 and the stomach wall 93. The gastrostomy tube 1 is fitted by inserting the internal retention device 3 (with the balloon 33 deflated) and internal end 21 of the first flexible tube 2 into the stoma tract of the patient to an appropriate depth (established using the gradated scale 26). A syringe containing sterile fluid is then connected to the third port 43 and fluid is inserted by pressing on the syringe to force fluid through the inflation channel 45, the secondary channel 24 and through an inflation hole 32 into the internal retention device 3 to inflate a balloon 33 (see Figure 5). The balloon 33 is attached to and positioned axially around a body 30 of the internal retention device 3. The attachment between the balloon 33 and the body 30 is airtight to prevent fluid escaping from the balloon 33 into the stomach. The first flexible tube 2 is then pulled until the inflated balloon 33 of the internal retention device 3 comes into contact with the stomach wall 93, as shown in Figure 9. The inflated balloon 33 of the internal retention device 3 thus protects the gastrostomy tube 1 from being prematurely or inadvertently pulled out of the stoma tract.

Once the internal retention device 3 is positioned against the stomach wall 93 as shown in Figure 9, the external retention device 7 is slid along the first flexible tube 2 until the underside 71 of the main body 70 contacts the surface of the skin 90. The first flexible tube 2 is then pushed through the elongate slot into the duct 74. The locking arm 72 is moved into the locked position so that the tooth 77 grips the first flexible tube 2 and prevents further longitudinal movement of the external retention device 7 relative to the first flexible tube 2. The external retention device 7 serves to prevent the gastrostomy tube 1 from being inadvertently pushed further inwards through the abdomen which could cause pain or discomfort to the patient or damage the stoma tract or the internal retention device 3. The internal retention device 3 and the external retention device 7 together prevent inadvertent longitudinal movement of the gastrostomy tube 1.

Once the gastrostomy tube 1 is in position, a nutrient or medicine source can be connected to the feed connection port 6. A nutrient or medicine can then be delivered into the stomach through the delivery channel along the entire length of the gastrostomy tube 1.

Where a nutrient or medicine source needs to be attached to the feed connection port 6, removed or changed, the clamp 8 around the second flexible tube 5 is first moved into the locked position to block the delivery channel. This prevents any backflow of stomach acids out through the feed connection port 6. By providing the clamp 8 on the single lumen second flexible tube 5 (rather than the dual lumen tube as in the prior art), the risk of damage to the separating wall 25 of the first flexible tube 2 is reduced. This reduces the overall failure rate of the gastrostomy tube 1 compared to existing tubes. During periods where no nutrient or medicine is required, the clamp 8 is typically retained in an open position to prevent permanent deformation or damage of the second flexible tube 5. In this situation the cap 65 can be screwed into the male part 63 of the feed connection port 6. This provides a seal to prevent backflow and leakage of stomach acids.

During use, it may be necessary to replace the feed connection port 6, for example to enable attachment of a nutrient or medicine source with a differently shaped connector, or because of damage to the feed connection port 6. In this case, the feed connection port can be replaced by simply cutting the second flexible tube 5 between the clamp 8 and the feed connection port 6 (when the clamp 8 is in the locked position). A new feed connection port 6 can then be fitted to the cut end of the second flexible tube 5.

It may also be necessary to use the gastrostomy tube 1 to drain the contents of a patient's stomach. This can be achieved by simply removing the nutrient or medicine source from the male port 63 of the feed connection port 6 (or opening the cap 65). With the clamp 8 open, the contents can then be drained through the gastrostomy tube 1. If rapid draining is required then the second flexible tube 5 can be cut to remove the feed connection port 6 entirely.

## Claims

1. A gastrostomy tube (1) for enteral nutrition or medication of a subject, comprising
a first flexible tube (2) having an internal end positionable *in vivo* within the stomach of the subject and an external end positionable *ex vivo,* wherein the first flexible tube has a main longitudinal channel (23) and a secondary longitudinal channel (24), and wherein the main and secondary longitudinal channels are not in fluid communication with each other;
an internal retention device (3) attached at or towards the internal end of the first flexible tube and in fluid communication with the secondary longitudinal channel;
a substantially Y-shaped connector (4) attached to the external end of the first flexible tube, wherein the substantially Y-shaped connector has a first port (41), a second port (42) and a third port (43), wherein the external end of the first flexible tube is connected to the first port, the second port is in fluid communication with the main longitudinal channel and the third port is in fluid communication with the secondary longitudinal channel; and
a second flexible tube (5) having a first end (52) and a second end (53) and a conduit extending between the first end and the second end, wherein the first end is bonded with a silicone adhesive to the second port of the substantially Y-shaped connector to define a delivery channel extending between the internal end of the first flexible tube and the second end of the second flexible tube for delivering a nutrient or medicine to the subject, wherein the second end of the second flexible tube is attachable to a nutrient or medicine source.

2. The gastrostomy tube of claim 1, further comprising a feed connection port attached to the second end of the second flexible tube.

3. The gastrostomy tube of claim 1 or 2, further comprising an external retention device adjustably mounted on the first flexible tube.

4. The gastrostomy tube of any preceding claim, wherein the first flexible tube and the substantially Y-shaped connector are composed of a first material and the second flexible tube is composed of a second material which is different from the first material.

5. The gastrostomy tube of claim 4, wherein the first flexible tube and the substantially Y-shaped connector are formed as a single continuous part.

6. The gastrostomy tube of any preceding claim, wherein a cross-sectional area of the conduit is the same size or larger than a cross-sectional area of the main longitudinal channel.

7. The gastrostomy tube of any preceding claim, wherein the main longitudinal channel has a substantially circular, oval or substantially D-shaped cross-section.

8. The gastrostomy tube of any preceding claim, wherein the internal retention device comprises a balloon having an elastic body with an interior void in fluid communication with the secondary longitudinal channel.

9. The gastrostomy tube of any preceding claim, wherein the second flexible tube is at least 5 cm in length.

10. A kit of parts comprising:
a gastrostomy tube according to any one of claims 1-9; and
a nutrient/medicine container attachable or attached to the gastrostomy tube via the second flexible tube or the feed connection port.

11. The kit of parts of claim 10, further comprising a first syringe attachable to the third port to supply fluid to or to remove fluid from the internal retention device.

12. The kit of parts of claim 11, wherein the first syringe is preloaded with a predetermined quantity of sterile fluid.

13. The kit of parts of any of claims 10-12 further comprising a second syringe attachable to the delivery channel via the second flexible tube or the feed connection port.

## Patentansprüche

1. Eine Gastrostomie-Sonde (1) für die enterale Ernährung oder Medikation eines Individuums, die Folgendes beinhaltet:
eine erste biegsame Sonde (2), die ein inneres Ende aufweist, das *in vivo* innerhalb des Magens des Individuums positionierbar ist, und ein äußeres Ende, das *ex vivo* positionierbar ist, aufweist, wobei die erste biegsame Sonde einen longitudinalen Hauptkanal (23) und einen longitudinalen sekundären Kanal (24) aufweist, und wobei der Hauptkanal und der längliche sekundäre Kanal nicht in Fluidkommunikation miteinander stehen;
eine innere Haltevorrichtung (3), die an dem inneren Ende der ersten biegsamen Sonde oder zu ihr hin angebracht ist und in Fluidkommunikation mit dem länglichen sekundären Kanal steht;
einen im Wesentlichen Y-förmigen Verbinder (4), der an dem äußeren Ende der ersten biegsamen Sonde angebracht ist, wobei der im Wesentlichen Y-förmige Verbinder einen ersten Anschluss (41), einen zweiten Anschluss (42) und einen dritten Anschluss (43) aufweist, wobei das äußere Ende der ersten biegsamen Sonde mit dem ersten Anschluss verbunden ist, der zweite Anschluss in Fluidkommunikation mit dem länglichen Hauptkanal steht und der dritte Anschluss in Fluidkommunikation mit dem länglichen sekundären Kanal steht; und
eine zweite biegsame Sonde (5), die ein erstes Ende (52) und ein zweites Ende (53) und eine Röhre, die sich zwischen dem ersten Ende und dem zweiten Ende erstreckt, aufweist, wobei das erste Ende mit einem Silikonhaftmittel an den zweiten Anschluss des im Wesentlichen Y-förmigen Verbinders gebunden ist, um einen Zufuhrkanal zu definieren, der sich zwischen dem inneren Ende der ersten biegsamen Sonde und dem zweiten Ende der zweiten biegsamen Sonde erstreckt, um einem Individuum einen Nährstoff oder ein Arzneimittel zuzuführen, wobei das zweite Ende der zweiten biegsamen Sonde an einer Nährstoff- oder Medizinquelle angebracht werden kann.

2. Gastrostomie-Sonde gemäß Anspruch 1, die ferner einen Einspeisungsverbindungsanschluss beinhaltet, der an dem zweiten Ende der zweiten biegsamen Sonde angebracht ist.

3. Gastrostomie-Sonde gemäß Anspruch 1 oder 2, die ferner eine äußere Haltevorrichtung beinhaltet, die verstellbar an der ersten biegsamen Sonde befestigt ist.

4. Gastrostomie-Sonde gemäß einem der vorhergehenden Ansprüche, wobei die erste biegsame Sonde und der im Wesentlichen Y-förmige Verbinder aus einem ersten Material bestehen und die zweite biegsame Sonde aus einem zweiten Material besteht, das sich von dem ersten Material unterscheidet.

5. Gastrostomie-Sonde gemäß Anspruch 4, wobei die erste biegsame Sonde und der im Wesentlichen Y-förmige Verbinder als ein einzelnes zusammenhängendes Teil gebildet sind.

6. Gastrostomie-Sonde gemäß einem der vorhergehenden Ansprüche, wobei eine Querschnittsfläche der Leitung gleich groß oder größer als eine Querschnittsfläche des länglichen Hauptkanals ist.

7. Gastrostomie-Sonde gemäß einem der vorhergehenden Ansprüche, wobei der längliche Hauptkanal einen im Wesentlichen kreisförmigen, ovalen oder im Wesentlichen D-förmigen Querschnitt aufweist.

8. Gastrostomie-Sonde gemäß einem der vorhergehenden Ansprüche, wobei die innere Haltevorrichtung einen Ballon beinhaltet, der einen elastischen Körper mit einem internen Hohlraum in Fluidkommunikation mit dem länglichen sekundären Kanal aufweist.

9. Gastrostomie-Sonde gemäß einem der vorhergehenden Ansprüche, wobei die zweite biegsame Sonde mindestens 5 cm lang ist.

10. Ein Teilesatz, der Folgendes beinhaltet:
eine Gastrostomie-Sonde gemäß einem der Ansprüche 1-9; und
einen Nährstoff-/Medizinbehälter, der über die zweite biegsame Sonde oder den Einspeisungsverbindungsanschluss an der Gastrostomie-Sonde angebracht werden kann oder an dieser angebracht ist.

11. Teilesatz gemäß Anspruch 10, der ferner eine erste Spritze beinhaltet, die an dem dritten Anschluss angebracht werden kann, um der inneren Haltevorrichtung Fluid zuzuleiten oder Fluid aus dieser zu entfernen.

12. Teilesatz gemäß Anspruch 11, wobei die erste Spritze mit einer vorbestimmen Menge an sterilem Fluid vorgeladen ist.

13. Teilesatz gemäß einem der Ansprüche 10-12, der ferner eine zweite Spritze beinhaltet, die über die zweite biegsame Sonde oder den Einspeisungsverbindungsanschluss an dem Zufuhrkanal angebracht werden kann.

## Revendications

1. Une sonde de gastrostomie (1) pour la nutrition ou la médication entérale d'un sujet, comprenant :
un premier tube flexible (2) ayant une extrémité interne pouvant être placée *in vivo* à l'intérieur de l'estomac du sujet et une extrémité externe pouvant être placée ex *vivo,* où le premier tube flexible a une voie longitudinale principale (23) et une voie longitudinale secondaire (24), et où les voies longitudinales principale et secondaire ne sont pas en communication fluidique l'une avec l'autre ;
un dispositif de retenue interne (3) rattaché au niveau de ou vers l'extrémité interne du premier tube flexible et en communication fluidique avec la voie longitudinale secondaire ;
un raccord substantiellement en Y (4) rattaché à l'extrémité externe du premier tube flexible, où le raccord substantiellement en Y a un premier orifice (41), un deuxième orifice (42) et un troisième orifice (43), où l'extrémité externe du premier tube flexible est raccordée au premier orifice, le deuxième orifice est en communication fluidique avec la voie longitudinale principale et le troisième orifice est en communication fluidique avec la voie longitudinale secondaire ; et
un deuxième tube flexible (5) ayant une première extrémité (52) et une deuxième extrémité (53) et un conduit s'étendant entre la première extrémité et la deuxième extrémité, où la première extrémité est collée à l'aide d'un adhésif en silicone au deuxième orifice du raccord substantiellement en Y afin de définir une voie d'apport s'étendant entre l'extrémité interne du premier tube flexible et la deuxième extrémité du deuxième tube flexible pour apporter un nutriment ou un médicament au sujet, où la deuxième extrémité du deuxième tube flexible peut être rattachée à une source de nutriment ou de médicament.

2. La sonde de gastrostomie de la revendication 1, comprenant en sus un orifice de raccordement pour aliments rattaché à la deuxième extrémité du deuxième tube flexible.

3. La sonde de gastrostomie de la revendication 1 ou de la revendication 2, comprenant en sus un dispositif de retenue externe monté de façon ajustable sur le premier tube flexible.

4. La sonde de gastrostomie de n'importe quelle revendication précédente, où le premier tube flexible et le raccord substantiellement en Y sont composés d'un premier matériau et le deuxième tube flexible est composé d'un deuxième matériau qui est différent du premier matériau.

5. La sonde de gastrostomie de la revendication 4, où le premier tube flexible et le raccord substantiellement en Y sont formés comme une seule pièce continue.

6. La sonde de gastrostomie de n'importe quelle revendication précédente, où une zone en coupe transversale du conduit est de taille identique ou plus grande qu'une zone en coupe transversale de la voie longitudinale principale.

7. La sonde de gastrostomie de n'importe quelle revendication précédente, où la voie longitudinale principale a une coupe transversale substantiellement circulaire, ovale ou conformée substantiellement en D.

8. La sonde de gastrostomie de n'importe quelle revendication précédente, où le dispositif de retenue interne comprend un ballonnet ayant un corps élastique avec un vide intérieur en communication fluidique avec la voie longitudinale secondaire.

9. La sonde de gastrostomie de n'importe quelle revendication précédente, où le deuxième tube flexible fait au moins 5 cm de long.

10. Un kit de pièces comprenant :
une sonde de gastrostomie selon l'une quelconque des revendications 1 à 9 ; et
un récipient pour nutriment/médicament qui peut être rattaché ou qui est rattaché à la sonde de gastrostomie via le deuxième tube flexible ou l'orifice de raccordement pour aliments.

11. Le kit de pièces de la revendication 10, comprenant en sus une première seringue pouvant être rattachée au troisième orifice afin d'introduire du fluide dans le dispositif de retenue interne ou de retirer du fluide de celui-ci.

12. Le kit de pièces de la revendication 11, où la première seringue est pré-remplie d'une quantité prédéterminée de fluide stérile.

13. Le kit de pièces de n'importe lesquelles des revendications 10 à 12 comprenant en sus une deuxième seringue pouvant être rattachée à la voie d'apport via le deuxième tube flexible ou l'orifice de raccordement pour aliments.
